(19) Europäisches Patentamt
European Patent Office
Office européen des brevets

(11) EP 0 664 286 B1

# (12) FASCICULE DE BREVET EUROPEEN

(45) Date de publication et mention de la délivrance du brevet:
**22.04.1998 Bulletin 1998/17**

(51) Int Cl.$^6$: **C07C 255/52**, C07C 251/48, C11B 9/00

(21) Numéro de dépôt: **95100485.2**

(22) Date de dépôt: **16.01.1995**

(54) **Nitriles tétraliniques, leur utilisation à titre d'ingrédients parfumants et intermédiaires oximes pour leur préparation**

Tetralinnitrile, ihre Verwendung als Inhaltsstoffe von Parfümen und Oximzwischenprodukte für ihre Herstellung

Tetralin nitriles, their use as ingredients for perfumes and oxime intermediates for their preparation

(84) Etats contractants désignés:
**CH DE FR GB LI NL**

(30) Priorité: **25.01.1994 CH 209/94**
**08.12.1994 CH 3714/94**

(43) Date de publication de la demande:
**26.07.1995 Bulletin 1995/30**

(73) Titulaire: **FIRMENICH SA**
**1211 Genève 8 (CH)**

(72) Inventeurs:
- **Fehr, Charles**
  **CH-1290 Versoix (CH)**
- **Delay, François**
  **CH-1227 Carouge (CH)**
- **Blanc, Pierre-Alain**
  **CH-1263 Crassier (CH)**
- **Chaptal-Gradoz, Nathalie**
  **CH-1202 Geneve (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 405 427**
**WO-A-93/13054**
**US-A- 5 087 770**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 664 286 B1

Printed by Jouve, 75001 PARIS (FR)

## Description

La présente invention a trait à l'industrie des parfums et plus particulièrement à un composé nouveau, à savoir le 5,6,7,8-tétrahydro-3,5,5,6,7,8,8-heptaméthyl-2-naphtalènecarbonitrile, sous forme de composé racémique et d'isomères énantiomériques, à savoir le (-)-(6S,7S)-5,6,7,8-tétrahydro-3,5,5,6,7,8,8-heptaméthyl-2-naphtalènecarbonitrile et le (+)-(6R,7R)-5,6,7,8-tétrahydro-3,5,5,6,7,8,8-heptaméthyl-2-naphtalènecarbonitrile.

L'invention a également pour objet des dérivés tétraliniques aldéhydés nouveaux employés à titre d'intermédiaires pour la préparation desdits isomères.

Le nitrile susmentionné est un analogue azoté du 5,6,7,8-tétrahydro-3,5,5,6,7,8,8-heptaméthyl-2-naphtalènecarbaldéhyde, un ingrédient parfumant à caractère musqué fort apprécié, décrit dans le brevet US 5,162,588. Nous avons maintenant découvert que l'utilisation en parfumerie des composés de l'invention peut se révéler fort avantageuse par rapport à celle dudit carbaldéhyde de l'art antérieur, notamment lors de l'emploi de ces composés dans des milieux agressifs tels que, par exemple, les produits de consommation du type savons, désodorisants et détergents.

Nous avons, en effet, constaté que le 5,6,7,8-tétrahydro-3,5,5,6,7,8,8-heptaméthyl-2-naphtalènecarbonitrile possède une odeur terreuse et musquée, dont la note de fond est dotée d'un caractère humique et terreux ressemblant beaucoup à l'odeur de son analogue aldéhydé cité plus haut. Quoique la note du composé racémique s'apparente à celle développée par ses deux énantiomères (-)-(6S,7S) et (+)-(6R,7R), le caractère olfactif de ces derniers se distingue du mélange racémique dans la mesure où le (-)-(6S,7S)-5,6,7,8-tétrahydro-3,5,5,6,7,8,8-heptaméthyl-2-naphtalènecarbonitrile possède une note plus puissante et boisée que celle de l'énantiomère (+)-(6R,7R).

D'un point de vue de la qualité de la note odorante, les composés de l'invention se révèlent donc tout aussi précieux et utiles que le carbaldéhyde connu et peuvent remplacer ce dernier dans ses applications typiques (voir par exemple US 5,162,588). Ceci est un résultat inattendu et fort avantageux dans la mesure où, comme il ressort des exemples présentés plus loin, les nitriles de l'invention sont chimiquement stables dans des milieux où leurs analogues aldéhydés peuvent présenter des problèmes de stabilité. Si l'on ajoute à cela le fait que ces nitriles se sont révélés encore plus tenaces sur des textiles que l'aldéhyde correspondant, on comprend la valeur des composés de l'invention, notamment pour le parfumage de détergents et adoucissants textiles.

Il convient de noter que la ressemblance et valeur olfactive comparable entre les aldéhydes et les nitriles observée dans le cas présent est un résultat inattendu au vu de l'art antérieur. C'est ainsi que lors d'une étude comparative entre analogues à fonction carboxyle ou nitrile, rapportée par R. De Simone dans Perfumer and Flavorist 4, 1 (1980), il a été constaté que, pour des composés à structure tétralinique ou indanique, les analogues nitriles de composés carboxylés à caractère fortement musqué pouvaient présenter des odeurs beaucoup plus faibles et même être pratiquement inodores. En particulier, le nitrile de la Tonalid® (origine : Polak's Frutal Works, USA), le céto-musc le plus intense connu à l'époque, s'était révélé avoir une odeur beaucoup plus faible que les nitriles d'autres composés cétoniques commerciaux que l'on savait moins intéressants que la Tonalid®. Il ressort ainsi de cette étude que la valeur olfactive du nitrile est imprévisible même lorsque l'on sait que son analogue carboxylé possède des propriétés intéressantes et utiles.

Les résultats de l'étude susmentionnée ont été confirmés postérieurement par la demanderesse, titulaire de la présente demande. Lors d'une étude exhaustive de dérivés tels qu'époxydes, alcools, esters, aldéhydes, méthyl cétones et nitriles de la Tonalid® et de la Célestolide® (origine : IFF Inc., USA), il a été constaté, lors de l'évaluation de ces dérivés par un panel d'experts parfumeurs, que les nitriles étaient olfactivement inférieurs aux aldéhydes, époxydes, et cétones correspondants, aussi bien en ce qui concernait la qualité que l'intensité de l'odeur, et ceci dans les deux séries de composés. En particulier, les parfumeurs ont trouvé que le nitrile de la Tonalid®, ayant la structure

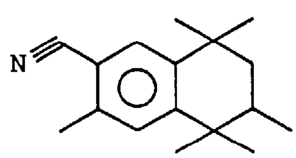

possédait une odeur très faiblement musquée, accompagnée d'une note nitrile et pharmaceutique.

Il est donc surprenant de constater que, malgré le fait que les composés de la présente invention et ses analogues décrits dans US 5,162,588 ne possèdent qu'un groupe méthyle de plus que la Tonalid® et ses analogues correspondants, leur comportement olfactif est tout à fait distinct de celui des composés connus, même en ce qui concerne la qualité et l'intensité relative de l'odeur à l'intérieur des deux familles de composés.

La présente invention fournit ainsi des nitriles nouveaux possédant des propriétés odorantes surprenantes par rapport aux composés connus de structure plus proche et comparables à celles de l'aldéhyde analogue connu et qui,

en plus, a un comportement parfaitement stable dans des milieux difficiles tels que savons, détergents et désodorisants. Par conséquent, ces nitriles peuvent être d'un emploi alternatif à celui dudit aldéhyde, emploi qui sera d'autant plus avantageux dans lesdits milieux difficiles.

De par sa structure, le 5,6,7,8-tétrahydro-3,5,5,6,7,8,8-heptaméthyl-2-naphtalènecarbonitrile peut se présenter sous deux formes isomériques de configuration cis et trans, dont l'isomère de configuration trans est préféré selon l'invention. Cependant, les mélanges d'isomères cis et trans sont également d'excellents ingrédients parfumants, produisant des effets olfactifs comparables.

A leur tour, ces deux isomères racémiques peuvent se présenter sous forme énantiomérique distincte. Comme indiqué plus haut, l'invention a plus particulièrement pour objet les deux énantiomères de formule

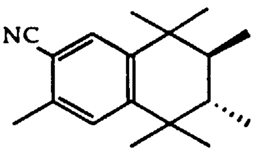

et

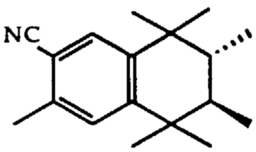

(-)-(6S,7S) (+)-(6R,7R)

Comme il a été cité précédemment, le 5,6,7,8-tétrahydro-3,5,5,6,7,8,8-heptaméthyl-2-naphtalènecarbonitrile soit sous forme racémique ou sous forme de l'un des énantiomères précités, peut être utilisé dans des conditions analogues à celles de l'usage du 5,6,7,8-tétrahydro-3,5,5,6,7,8,8-heptaméthyl-2-naphtalènecarbaldéhyde, aussi bien en ce qui concerne la nature des compositions dans lesquelles il peut être incorporé, que les proportions dans lesquelles il sera utilisé. Ces conditions sont décrites en détail dans US 5,162,588. D'autres applications du 5,6,7,8-tétrahydro-3,5,5,6,7,8,8-heptaméthyl-2-naphtalènecarbonitrile, qui mettent en évidence en particulier sa stabilité chimique et sa remarquable tenacité, sont présentés plus loin.

Bien entendu, quoique l'emploi alternatif des nitriles de l'invention soit plus avantageux pour le parfumage de savons, détergents et produits d'entretien, adoucissants textiles, désodorisants et antiperspirants, ces composés peuvent également être utilisés dans le parfumage d'autres articles de consommation tels que parfums et eaux de toilette, shampoings et autres produits d'hygiène capillaire, gels de douche ou bain et préparations cosmétiques.

Les composés selon l'invention peuvent être préparés par plusieurs méthodes. Selon l'invention, on préfère les préparer par réaction de l'oxime du 5,6,7,8-tétrahydro-3,5,5,6,7,8,8-heptaméthyl-2-naphtalènecarbaldéhyde avec un agent déshydratant, notamment l'anhydride acétique. Les conditions particulières de la réaction sont décrites plus loin. D'autres agents déshydratants peuvent bien entendu être employés. Une énumération de ces réactifs est ici superflue, ce type de réactions étant bien connu de l'homme de métier [voir, par exemple, J. March, Advanced Organic Chemistry : Reactions, Mechanisms and Structure, 3rd ed., section 7-40, John Wiley & Sons, USA (1985)].

Les oximes de départ dans ce procédé sont des composés nouveaux qui font également l'objet de l'invention. Elles peuvent être préparées à partir du carbaldéhyde correspondant à l'aide de techniques conventionnelles et dans les conditions décrites plus loin (voir également J. Chem. Soc. 1965,1564).

Lorsque l'on désire obtenir les isomères de configuration cis ou trans du nitrile de l'invention, on utilise en tant que produits de départ les isomères correspondants du carbaldéhyde susmentionné et de l'oxime subséquente. La préparation du 5,6,7,8-tétrahydro-3,5,5,6,7,8,8-heptaméthyl-2-naphtalènecarbaldéhyde, ainsi que de ses isomères de configuration cis et trans, est décrite en détail dans US 5,162,588.

La préparation des énantiomères aldéhydés correspondants (-)-(6S,7S) et (+)-(6R,7R) a été effectuée par un procédé particulier qui peut être illustré par le schéma que voici :

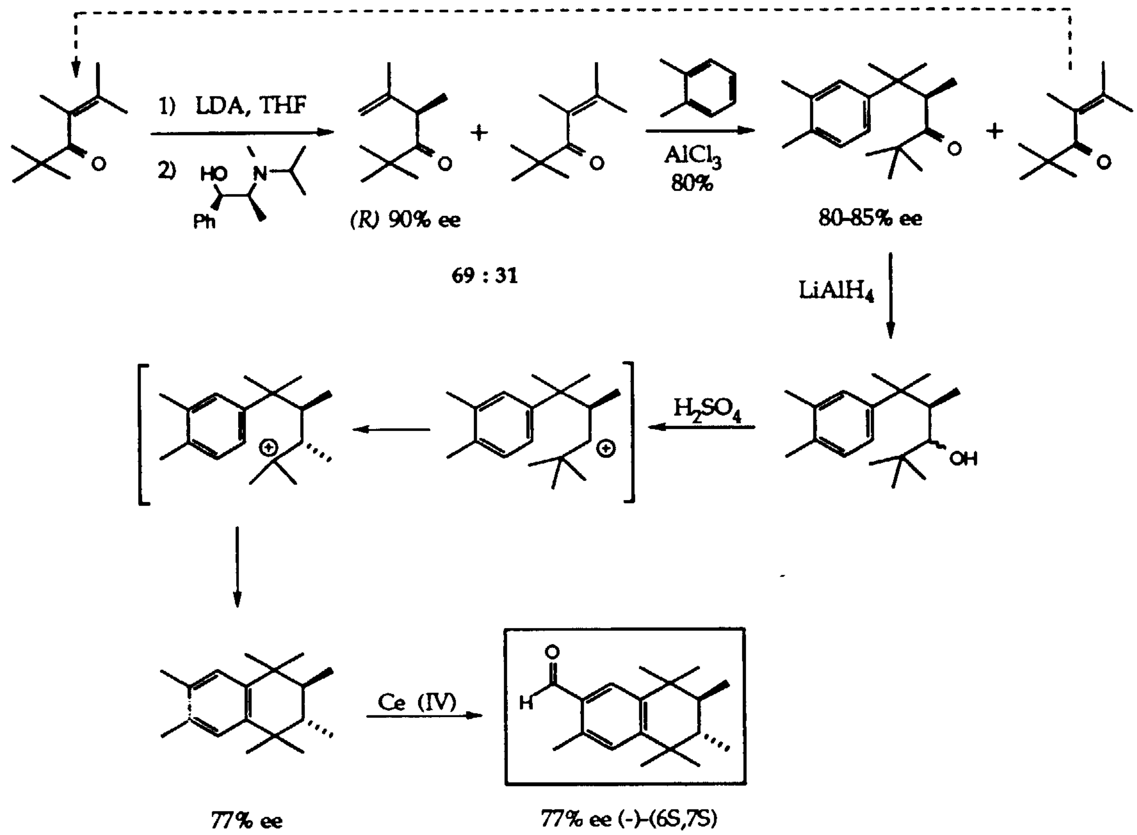


L'énantiomère (+)-(6R,7R) peut être obtenu de façon tout à fait analogue en remplaçant la (-)-N-isopropyléphédrine par la (+)-N-isopropyléphédrine. L'aldéhyde de configuration (+)-(6R,7R) avait une pureté optique égale à 82% ee.

Les composés aldéhydés précités sont non seulement des intermédiaires utiles pour la préparation des dérivés nitriles de l'invention, mais également des ingrédients parfumants utiles. En particulier, nous avons trouvé que le (-)-(6S,7S)-5,6,7,8-tétrahydro-3,5,5,6,7,8,8-heptaméthyl-2-naphtalènecarbaldéhyde était le composé qui possédait les propriétés odorantes les plus développées. Son caractère musqué, très terreux, légèrement ambré est particulièrement puissant. Il possède une note qui se différencie de celle montrée par le (+)-(6R,7R) dans le sens que ce dernier développe un caractère moins terreux, avec un côté boisé et dont la puissance est nettement inférieure. Leur différence olfactive sera montrée ci-après à l'aide d'exemples d'application comparative.

La présente invention a donc également pour objet lesdits composés aldéhydés ainsi que leur utilisation en tant qu'ingrédients parfumants pour la préparation de parfums ou bases parfumantes ou pour le parfumage d'articles de consommation divers.

La présente invention sera maintenant décrite de façon plus détaillée à l'aide des exemples suivants. Dans les exemples de préparation des composés de l'invention les températures sont indiquées en degrés centigrades et les abréviations ont le sens usuel dans l'art.

Exemple 1

Préparation du trans-5,6,7,8-tétrahydro-3,5,5,6,7,8,8-heptaméthyl-2-naphtalènecarbonitrile

a) Oxime de trans-5,6,7,8-tétrahydro-3,5,5,6,7,8,8-heptaméthyl-2-naphtalènecarbaldéhyde

On a chargé un ballon sous agitation avec 17,2 g (247,5 mmole) d'hydrochlorure d'hydroxylamine dans 110g d'eau. On a ensuite ajouté à cette solution 50g (193,8 mmole) de trans-5,6,7,8-tétrahydro-3,5,5,6,7,8,8-heptaméthyl-2-naphtalènecarbaldéhyde, 158 g de méthanol et 290 g de diisopropyléther. Finalement, une solution de carbonate de potassium (16,8 g, 121,7 mmole) dans 90 g d'eau a été introduite goutte à goutte sans que la température du mélange réactionnel dépasse 35°. Après environ 2 h de réaction, on a extrait à l'éther et concentré la phase organique pour obtenir 60 g de produit brut. Celui-ci a été recristallisé dans du cyclohexane pour fournir 33 g d'oxime de trans-5,6,7,8-tétrahydro-3,5,5,6,7,8,8-heptaméthyl-2-naphtalènecarbaldéhyde pure à 91% (rend.

72,9%).

| | |
|---|---|
| IR : | 3256, 2970, 1610, 1502, 1464, 1393, 1362, 1312, 1222, 1112, 976, 940, 898, 856 $cm^{-1}$ |
| RMN($^1H$, 360MHz) : | 0,97(d, J=6,5, 6H) ; 1,10(s, 6H) ; 1,32(s, 3H) ; 1,34(s, 3H) ; 1,59(m, 2H) ; 2,39(s, 3H) ; 7,17(s, 1H) ; 7,65(s, 1H) ; 7,93(s large, 1H échange avec $D_2O$) ; 8,36(s, 1H) δ ppm |
| RMN($^{13}C$, 360MHz) : | 13,8(2q) ; 19,6(q) ; 25,4(q) ; 25,6(q) ; 29,4(q) ; 29,5(q) ; 37,7(s) ; 37,9(s) ; 39,2(d) ; 39,3 (d) ; 125,9(d) ; 127,7(s) ; 129,6(d) ; 135,5(s) ; 143,9(s) ; 147,9(s) ; 149,6(d) δ ppm |
| SM : | 273(20, $M^+$), 258(41), 240(13), 216(37), 202(100), 197(56), 184(88), 170(13), 156(16), 142(14), 128(12), 115(15), 91(11), 77(8), 69(5), 57(75), 43(37). |

b) On a chauffé à 120°, pendant 1 h, 76 g (0,745 mole) d'anhydride acétique et ajouté ensuite, pendant 4 $^1/_2$ h, une solution de l'oxime brute préparée selon a) (136,5 g ; ~ 0,5 mole) dans 1500 ml (1573 g) d'acide acétique. Après concentration du mélange de la réaction, on a obtenu 281 g d'un produit brut partiellement solide, de couleur foncée. On a repris dans 400 g de cyclohexane et filtré le précipité. Afin d'éliminer complètement l'acide acétique, on a repris le solide dans $CHCl_3$ et lavé avec $NaHCO_3$. Après concentration du produit ainsi traité, on a obtenu 64,5g d'un produit pur à 92,1%. Ce produit a été purifié encore par cristallisation dans le cyclohexane pour fournir le trans-5,6,7,8-tétrahydro-3,5,5,6,7,8,8-heptaméthyl-2-naphtalènecarbonitrile à 98,5% pur.

| | |
|---|---|
| IR : | 2969, 2873,5, 2223, 1610, 1500, 1474,5, 1392, 1364, 1220, 1112, 1025, 926, 866 $cm^{-1}$ |
| RMN($^1H$, 360MHz) : | 0,97(d, J=6,5,6H) ; 1,05(s, 3H) ; 1,07(s, 3H) ; 1,28(s, 3H) ; 1,29(s, 3H) ; 1,56(m, 2H) ; 2,47(s, 3H) ; 7,25(s, 1H) ; 7,59(s, 1H) δ ppm |
| RMN($^{13}C$, 360MHz) : | 13,7(q) ; 13,8(q) ; 20,0(q) ; 25,3(q) ; 25,6(q) ; 29,2(q) ; 29,4(q) ; 37,7(s) ; 38,3(s) ; 39,0 (2d) ; 110,1(s) ; 118,8(s) ; 129,0(d) ; 132,0(d) ; 137,7(s) ; 144,3(s) ; 151,2(s) δ ppm |
| SM : | 255(9, $M^+$), 240(22), 198(55), 184(100), 168(6), 156(10), 140(5), 127(5), 115(6), 57(38), 43(21). |

Quoiqu'on ait décrit ci-dessus la préparation de l'isomère trans, la même méthode peut être appliquée à la préparation de l'autre isomère ou de mélanges des deux.

Exemple 2

Préparation de (-)-(6S,7S)- et de (+)-(6R,7R)-5,6,7,8-tétrahydro-3,5,5,6,7,8,8-heptaméthyl-2-naphtalènecarbonitrile

a) 2,2,4,5-Tétraméthyl-4-hexén-3-one
30,8 g (0,2 mole) de 2,2,4,5-tétraméthyl-5-hexén-3-one ont été chauffés à 100° dans 100 ml de toluène en présence de 3,08 g d'acide p-toluènesulfonique. Après 24 h, le mélange a été refroidi et extrait à l'éther. Après neutralisation avec une solution aqueuse saturée de $NaHCO_3$, la phase organique a été lavée à l'eau, avec une solution saturée de NaCl puis séchée avec du $Na_2SO_4$.
Par évaporation et distillation du résidu suivies de distillation fractionnée on a obtenu une fraction ayant Eb. 60°/ 1,06 x $10^3$ Pa
RMN($^1H$, 360MHz) : 1,18(s, 9H) ; 1,57(s, 3H) ; 1,66(s, 3H) ; 1,77(s, 3H) δ ppm
SM : m/z : 154(3, $M^+$), 97(100), 69(62), 41(65), 39(20).

b) (-)-(R)-2,2,4,5-Tétraméthyl-5-hexén-3-one
Une solution de 15,4g (100 mmole) de la cétone obtenue comme indiqué sous lettre a) dans 80 ml de tétrahydrofuranne (THF) a été ajoutée en 30 min à -45° à 200 mmole de lithiumdiisopropylamide dans 150 ml de THF.
Une solution de 53,8 g (260 mmole) de (-)-N-isopropyléphédrine dans 250 ml de THF a été ajoutée au mélange en 1 h à -45°, puis le tout a été porté à -10° en l'espace de 20 min.
Le mélange a été ensuite versé sous vigoureuse agitation dans une solution aqueuse à 20% de HCl et extrait 3 fois avec de l'éther. La phase organique a été lavée successivement avec du bicarbonate, de l'eau et de la saumure, puis séchée sur $Na_2SO_4$ et concentrée sous pression réduite. Le résidu (17,6 g) a été employé sans purification ultérieure. Un échantillon du résidu obtenu a été purifié par chromatographie sur colonne ($SiO_2$) par élution au pentane:éther (19:1) pour fournir un produit ayant 90% ee $[\alpha]^{20}_D$ = -235° (c = 2,2 ; $CHCl_3$).

Les données analytiques étaient identiques à celles décrites pour un échantillon racémique [Helv. Chim. Acta 1989, 72, 1537].

En remplaçant la (-)-N-isopropyléphédrine par la (+)-N-isopropyléphédrine et en opérant comme indiqué ci-dessus, on a obtenu le (+)-(S)-2,2,4,5-tétraméthyl-5-hexén-3-one avec 90% ee.

c) (-)-(R)-5-(3,4-Diméthyl-phényl)-2,2,4,5-tétraméthyl-hexan-3-one

En opérant comme indiqué dans Helv. Chim. Acta 1989, 72, 1537, on a traité à 0° le produit obtenu sous lettre b) avec du trichlorure d'aluminium (1,2 éq. pour 1 éq. de composé cétonique) dans de l'oxylène. Après extraction et évaporation du solvant, on a obtenu une huile (20,8g) qui par distillation fractionnée a fourni 15,3 g (rend. 61%) de la cétone désirée.

$[\alpha]^{20}_D$ = -102°(c = 2,0 ; $CHCl_3$).

Les caractères spectroscopiques étaient identiques à ceux décrits pour le composé racémique [Helv. Chim. Acta, op. cit.].

De façon analogue on a obtenu l'isomère (+)-(S) correspondant

$[\alpha]^{20}_D$ = +109° (c = 2,2 ; $CHCl_3$).

d) (-)-(2S,3S)-1,2,3,4-Tétrahydro-1,1,2,3,4,4,6,7-octaméthylnaphtalène

On a effectué la réduction des composés obtenus sous lettre c) en utilisant de l'aluminohydrure de lithium dans l'éther comme indiqué dans Helv. Chim. Acta, op. cit.

Le produit obtenu a été cyclisé soit par traitement avec de l'acide méthanesulfonique et $P_2O_5$, soit avec de l'acide sulfurique (15 g) dans 20 ml de $CH_2Cl_2$ à -10° pour 15 min. Une extraction à l'éther suivie de cristallisation à l'alcool éthylique a fourni 4,92g du composé désiré et une huile ayant une teneur en ce même composé de 2,8g.

$[\alpha]^{20}_D$ = -37° (c = 0,8 ; $CHCl_3$).

L'isomère (+) correspondant a été obtenu de façon analogue.

$[\alpha]^{20}_D$ = +40° (c = 1,0 ; $CHCl_3$).

Les caractères spectroscopiques étaient identiques à ceux décrits pour le composé racémique [Helv. Chim. Acta, op. cit.].

e) (-)-(6S,7S)-5,6,7,8-Tétrahydro-3,5,5,6,7,8,8-heptaméthyl-2-naphtalènecarbaldéhyde

En opérant comme indiqué dans Helv. Chim. Acta, op. cit., on a oxydé à l'aide de $Ce(NH_4)_2(NO_3)_6$ le composé obtenu sous lettre d). Pour ce faire on a opéré dans le méthanol à 50°.

Le produit obtenu a été purifié par chromatographie sur colonne ($SiO_2$) par élution au pentane:éther (97:3) pour fournir 2,0 g du naphtalènecarbaldéhyde avec un rendement de 61%.

Un échantillon a été recristallisé dans l'alcool éthylique.

$[\alpha]^{20}_D$ = -39° (c = 0,85 ; $CHCl_3$) ; env. 77% ee

En opérant de façon analogue on a obtenu le composé (+) correspondant

$[\alpha]^{20}_D$ = +42° (c = 1,08 ; $CHCl_3$) ; env. 82% ee.

Les caractères spectroscopiques étaient en tout point identiques à ceux décrits pour le composé racémique correspondant [Helv. Chim. Acta, op. cit.].

f) (-)-(6S,7S)-5,6,7,8-Tétrahydro-3,5,5,6,7,8,8-heptaméthyl-2-naphtalènecarbonitrile et son isomère (+)-(6R,7R)-correspondant

En opérant de manière analogue à celle indiquée par T. van Es [J. Chem. Soc., 1965, 1564] et en utilisant comme produits de départ les deux énantiomères du naphtalènecarbaldéhyde obtenus sous lettre e), on a obtenu les carbonitriles correspondants.

Ainsi 0,800 g (3,10 mmole) de (-)-(6S,7S)-5,6,7,8-tétrahydro-3,5,5,6,7,8,8-heptaméthyl-2-naphtalènecarbaldéhyde ont été mélangés avec 0,280 g (4 mmole) d'hydrochlorure d'hydroxylamine, 0,420 g de formiate de sodium (6,20 mmole) et 5 ml d'acide formique et le tout a été chauffé à reflux pendant 45 min.

Après refroidissement à 30°, on a hydrolysé avec de l'eau et extrait 3 fois à l'éther. Après les traitements usuels de séparation de la phase organique, lavage avec $NaHCO_3$ et NaCl suivis de séchage sur $Na_2SO_4$, on a traité la solution éthérée avec du charbon actif (0,200 g).

La solution a été ainsi chauffée à reflux pendant 1/4 h, puis concentrée.

Le résidu obtenu a été purifié par élution au cyclohexane:acétate d'éthyle (98:2) sur une colonne de $SiO_2$ (F 60 ; 40-60μ ; 40 g).

Par évaporation du solvant on a recueilli 0,620 g d'un solide jaune qui par recristallisation (2 fois avec un mélange 1:1 d'éther et de cyclohexane) a fourni 0,360g de (-)-(65,7S)-5,6,7,8-tétrahydro-3,5,5,6,7,8,8-heptaméthyl-2-naphtalènecarbonitrile ayant

$[\alpha]^{20}_D$ = -39° (c = 1,1 ; $CHCl_3$) ; env. 77% ee.

En opérant de façon analogue mais en utilisant comme produit de départ le (+)-(6R,7R)-5,6,7,8-tétrahydro-3,5,5,6,7,8,8-heptaméthyl-2-naphtalènecarbaldéhyde on a obtenu le nitrile correspondant ayant

$[\alpha]^{20}_D$ = +45° (c = 0,94 ; $CHCl_3$) ; env. 82% ee.

Exemple 3

Test de stabilité dans une composition antiperspirante pour roll-on

Une composition antiperspirante, destinée à être appliquée à l'aide d'un dispositif de type roll-on, a été préparée par mélange des ingrédients suivants :

| Ingrédients | | % en poids |
|---|---|---|
| A. | Natrosol 250 H 1) | 0,70 |
| | Eau distillée | 39,80 |
| B. | Locron L 2) | 20,00 |
| | 1,3-Butylène-glycol | 2,00 |
| | Ethanol 95° | 35,00 |
| C. | Parfum 3) | 1,00 |
| | Chremophor RH 40 4) | 1,50 |
| | Total | 100,00 |

1) hydroxyéthylcellulose, Hercules Co.
2) hydroxychlorure d'aluminium, Hoechst AG
3) trans-5,6,7,8-tétrahydro-3,5,5,6,7,8,8-heptaméthyl-2-naphtalènecarbonitrile, solution à 10% dans le myristate d'isopropyle (MIP)
4) huile de ricin hydrogéné et éthoxylé, BASF AG

On a répandu le Natrosol dans l'eau sous agitation vigoureuse jusqu'à obtenir un gel transparent.
Les ingrédients de la partie B ont ensuite été mélangés entre eux et ce mélange a été versé dans la partie A. La partie C a été ajoutée par la suite et le mélange transparent obtenu était alors prêt pour être versé dans des dispositifs de type roll-on.
Ce mélange qui développait l'odeur musquée, terreuse caractéristique du composé de l'invention, a été testé pendant 1 mois, à 22°C et à 40°C, pour la stabilité de l'odeur développée.

A la fin de la période de test, un panel d'experts parfumeurs a jugé que l'odeur était restée inchangée, aussi bien du point de vue de son caractère que de son intensité.

Exemple 4

Test de stabilité dans une composition antiperspirante pour spray aérosol

Une composition antiperspirante, destinée à être appliquée à l'aide d'un distributeur de type "spray", a été préparée par mélange des ingrédients suivants :

| Ingrédients | | % en poids |
|---|---|---|
| I. | Cyclométhicone 1) | 51,00 |
| | Parfum 2) | 4,00 |
| | Isopropyl myristate | 8,75 |
| | Aerosil 200 3) | 1,00 |
| | Bentone 38 4) | 3,25 |
| | Chlorhydrate d'aluminium 5) | 32,00 |
| | Total | 100,00 |

1) huile de silicone volatile DC 344 et DC 345, à parties égales ; origine : DOW Chemicals, USA
2) voir Exemple 1
3) origine : Degussa
4) quaternium 18-hectorite ; origine : NL Industries
5) par exemple, Mico Dry Ultrafine ; origine : Reheis Chem. Corp., USA

On a mélangé la partie I et ensuite ajouté la Bentone 38 et l'Aérosil 200. On a agité pendant quelques minutes à l'aide d'un agitateur à haute vitesse (par exemple, du type Ultra-Turray, Homorex, Silverson) jusqu'à ce que le mélange soit aussi épais que possible. Finalement, on a ajouté le chlorhydrate d'aluminium sous agitation. Le tout a été bien mélangé et ensuite versé dans des récipients aérosol, à raison de 25% du mélange ainsi préparé pour 75% de Drivosol 27 (propulseur ; origine : Hüls).

Le produit ainsi obtenu a été testé dans les conditions décrites à l'Exemple 3. De l'avis du panel de parfumeurs qui a évalué le produit après le mois de test, aussi bien l'aspect que l'odeur de celui-ci étaient restés parfaitement inchangés.

Exemple 5

Test de substantivité du linge

On a ajouté à une base non parfumée d'adoucissant textile du commerce, d'une part, 0,1% de 5,6,7,8-tétrahydro-3,5,5,6,7,8,8-heptaméthyl-2-naphtalènecarbonitrile et, d'autre part, 0,1% de 5,6,7,8-tétrahydro-3,5,5,6,7,8,8-heptaméthyl-2-naphtalènecarbaldéhyde.
Deux lots standard de textiles ont ensuite été traités avec ces deux échantillons d'adoucissant textile parfumés lors d'un cycle de lavage dans des machines à laver le linge séparées, mais opérant dans les mêmes conditions.
Les deux lots de textiles ont ensuite été évalués à l'aveugle par un panel de 4 experts parfumeurs, à la sortie de la machine, après le séchage et une semaine plus tard.
De l'avis des parfumeurs, les deux lots de textiles développaient la même odeur, aussi bien à la sortie de la machine, qu'après leur séchage.
Après une semaine, les textiles traités avec l'adoucissant textile contenant le 5,6,7,8-tétrahydro-3,5,5,6,7,8,8-heptaméthyl-2-naphtalènecarbonitrile ont été unanimement choisis par les parfumeurs, de par la qualité et l'intensité de leur odeur musquée, pratiquement inchangée en caractère et intensité depuis leur traitement, alors que l'odeur de l'autre lot de textiles, quoique toujours bien perceptible, était déjà plus faible.

Exemple 6

Eau de toilette masculine

On a préparé une eau de toilette masculine en mélangeant les ingrédients suivants (parties en poids) :

| Ingrédients | Parties en poids |
|---|---|
| Acétate de linalyle | 200 |

(suite)

| Ingrédients | Parties en poids |
|---|---|
| Allylamyl glycolate | 80 |
| Bergamote | 500 |
| Linalol | 150 |
| Citral | 30 |
| α-Damascone 10% * 1) | 70 |
| Dihydromyrcenol | 900 |
| Polysantol® * 1) 3) | 50 |
| Essence de galbanum 10% * | 70 |
| Genièvre synthétique | 60 |
| Géranium Chine | 40 |
| Héliopropanal 10% * | 50 |
| Hédione® * 1) 4) | 230 |
| Iralia® 1) 5) | 100 |
| Iso E Super 2) 6) | 130 |
| Labdanum Ciste 10% * | 50 |
| Essence de lavandin | 150 |
| Lyral® 2) 7) | 100 |
| Menthe du Brésil | 15 |
| Méthylnaphtylcétone | 10 |
| Mousse cristal | 30 |
| Muscade | 50 |
| Octynecarbonate de méthyle 1% * | 35 |
| Essence d'orange du Portugal | 100 |
| Précyclémone ® B 2) 8) | 70 |
| Sauge sclarée | 80 |
| Vertofix ® coeur 2) 9) | 300 |
| Zestover 10% * 1) 10) | 70 |
| Galbex® 183 * 1) | 200 |
| Rose essence 193 D * 1) | 20 |
| Cetalox® 1) 11) | 10 |
| Total | 3950 |

* dans le dipropylèneglycol
1) origine: Firmenich SA, Genève, Suisse
2) origine: IFF (International Flavors & Fragrances, USA)
3) 3,3-diméthyl-5-(2',2',3'-triméthyl-3'-cyclopentén-1'-yl)-4-pentén-2-ol
4) 3-oxo-2-pentyl-cyclopentanacétate de méthyle
5) méthylionone
6) 2,3,8,8,-tétraméthyl-2-acétyl-1,2,3,4,5,6,7,8-octahydronaphtalène
7) 4-(4-hydroxy-4-méthylpentyl)-3-cyclohexène-1-carboxaldéhyde
8) 1-méthyl-4-(4-méthyl-3-pentényl)-3-cyclohexène-1-carboxaldéhyde
9) dérivés cétoniques de l'essence de cèdre du Texas
10) 2,4-diméthyl-3-cyclohexène-1-carbaldéhyde
11) dodécahydro-3a,6,6,9a-tétraméthyl-naphto[2.1b]furanne

En ajoutant à cette composition 50 parties en poids de (-)-(6S,7S)-5,6,7β-tétrahydro-3,5,5,6,7,8,8-heptaméthyl-2-naphtalènecarbaldéhyde on a obtenu une nouvelle composition ("test") dont le caractère musqué et ambré était nettement plus marqué que celui de la composition de base. Par comparaison à l'aveugle avec une nouvelle composition contenant une quantité identique de l'énantiomère (+)-(6R,7R), la composition test a été trouvée plus puissante par un panel de huit parfumeurs.

## Revendications

1. 5,6,7,8-Tétrahydro-3,5,5,6,7,8,8-heptaméthyl-2-naphtalènecarbonitrile sous forme racémique ou sous forme de l'un de ses isomères optiquement actifs.

2. Trans-5,6,7,8-tétrahydro-3,5,5,6,7,8,8-heptaméthyl-2-naphtalènecarbonitrile.

3. (-)-(6S,7S)-5,6,7,8-Tétrahydro-3,5,5,6,7,8,8-heptaméthyl-2-naphtalènecarbonitrile.

4. (+)-(6R,7R)-5,6,7,8-Tétrahydro-3,5,5,6,7,8,8-heptaméthyl-2-naphtalènecarbonitrile.

5. Utilisation du 5,6,7,8-tétrahydro-3,5,5,6,7,8,8-heptaméthyl-2-naphtalènecarbonitrile sous forme racémique ou sous forme de l'un de ses isomères optiquement actifs, à titre d'ingrédient parfumant.

6. Composition parfumante ou article parfumé contenant à titre d'ingrédient actif le 5,6,7,8-tétrahydro-3,5,5,6,7,8,8-heptaméthyl-2-naphtalènecarbonitrile sous forme racémique ou sous forme de l'un de ses isomères optiquement actifs.

7. Article parfumé selon la revendication 6, sous forme d'un parfum ou d'une eau de toilette, d'un savon, d'un gel de douche ou bain, d'un shampoing ou autre produit pour les cheveux, d'une préparation cosmétique, d'un désodorisant corporel ou d'air ambiant, d'un détergent ou adoucissant textile, ou d'un produit d'entretien.

8. Procédé pour la préparation du 5,6,7,8-tétrahydro-3,5,5,6,7,8,8-heptaméthyl-2-naphtalènecarbonitrile, caractérisé en ce qu'on fait réagir l'oxime de 5,6,7,8-tétrahydro-3,5,5,6,7,8,8-heptaméthyl-2-naphtalènecarbaldéhyde avec un agent déshydratant.

9. Procédé selon la revendication 8, caractérisé en ce qu'on utilise comme produit de départ l'oxime de trans-5,6,7,8-tétrahydro-3,5,5,6,7,8,8-heptaméthyl-2-naphtalènecarbaldéhyde pour obtenir le trans-5,6,7,8-tétrahydro-3,5,5,6,7,8,8-heptaméthyl-2-naphtalènecarbonitrile.

10. Oxime de 5,6,7,8-tétrahydro-3,5,5,6,7,8,8-heptaméthyl-2-naphtalènecarbaldéhyde.

11. (-)-(6S,7S)-5,6,7,8-Tétrahydro-3,5,5,6,7,8,8-heptaméthyl-2-naphtalènecarbaldéhyde.

12. (+)-(6R,7R)-5,6,7,8-Tetrahydro-3,5,5,6,7,8,8-heptaméthyl-2-naphtalènecarbaldéhyde.

13. Utilisation des composés des revendications 11 et 12 à titre de produits de départ pour la préparation de (-)-(6S, 7S)-5,6,7,8-tétrahydro-3,5,5,6,7,8,8-heptaméthyl-2-naphtalènecarbonitrile et (+)-(6R,7R)-5,6,7,8-tétrahydro-3,5,5,6,7,8,8-heptaméthyl-2-naphtalènecarbonitrile, caractérisée en ce qu'on transforme, de façon connue en soi, lesdits produits de départ en l'oxime correspondante et en ce qu'on fait réagir cette dernière avec un agent déshydratant.

14. Utilisation des composés des revendications 11 et 12 en tant qu'ingrédients parfumants, pour la préparation de parfums ou bases parfumantes et pour le parfumage d'articles de consommation.

## Patentansprüche

1. 5,6,7,8-Tetrahydro-3,5,5,6,7,8,8-heptamethyl-2-naphthalincarbonitril als Racemat oder in Form eines seiner optisch aktiven Isomeren.

2. Trans-5,6,7,8-Tetrahydro-3,5,5,6,7,8,8-heptamethyl-2-naphthalincarbonitril.

3. (-)-(6S,7S)-5,6,7,8-hydro-3,5,5,6,7,8,8-heptamethyl-2-naphthalincarbonitril.

4. (+)-(6R,7R)-5,6,7,8-Tetrahydro-3,5,5,6,7,8,8-heptamethyl-2-naphthalincarbonitril.

5. Verwendung eines Racemats oder eines optisch aktiven Isomeren von 5,6,7,8-Tetrahydro-3,5,5,6,7,8,8-heptamethyl-2-naphthalincarbonitril als Riechstoff

6. Parfümzusammensetzung oder parfümierter Artikel, enthaltend als aktiven Bestandteil 5,6,7,8-Tetrahydro-3,5,5,6,7,8,8-heptamethyl-2-naphthalincarbonitril als Racemat oder in Form eines seiner optisch aktiven Isomeren.

7. Parfümierter Artikel nach Anspruch 6 in Form eines Parfüms oder eines Eau de Toilette, einer Seife, eines Dusch- oder Badegels, eines Shampoos oder eines anderen Haarpflegeartikels, eines kosmetischen Präparats, eines Körperdeodorants oder eines Raumlauftverbesserers, eines Detergens oder Textilweichspülers, oder eines Universalhaushaltsreinigers.

8. Verfahren zur Herstellung von 5,6,7,8-Tetrahydro-3,5,5,6,7,8,8-heptamethyl-2-naphthalincarbonitril, dadurch gekennzeichnet, daß das Oxim von 5,6,7,8-Tetrahydro-3,5,5,6,7,8,8-heptamethyl-2-naphthalincarbaldehyd mit einem Dehydratisierungsreagens zur Reaktion gebracht wird.

9. Verfahren nach Anspruch 8, dadurch gekennzeichnet, daß als Ausgangsprodukt das Oxim von trans-5 6,7,8-Tetrahydro-3,5,5,6,7,8,8-heptamethyl-2-naphthalincarhaldehyd verwendet wird, um trans-5,6,7,8-Tetrahydro-3,5,5,6,7,8,8-heptamethyl-2-naphthalincarbonitril zu erhalten.

10. Das Oxim von 5,6,7,8-Tetrahydro-3,5,5,6,7,8,8-heptamethyl-2-naphthalincarbaldehyd.

11. (-)-(6S,7S)-5,6,7,8-Tetrahydro-3,5,5,6,7,8,8-heptamethyl-2-naphthalincarbaldehyd.

12. (+)-(6R,7R)-5,6,7,8-Tetrahydro-3,5,5,6,7,8,8-heptamethyl-2-naphthalincarbaldehyd.

13. Verwendung der Verbindungen nach den Ansprüchen 11 und 12 als Ausgangsverbindungen zur Herstellung von (-)-(6S,7S)-5,6,7,8-Tetrahydro-3,5,5,6,7,8,8-heptamethyl-2-naphthalincarbonitril und (+)-(6R,7R)-5,6,7,8-Tetrahydro-3,5,5,6,7,8,8-heptamethyl-2-naphthalincarbonitril, dadurch gekennzeichnet, daß man in an sich bekannter Weise die genannten Ausgangsverbindungen in das entsprechende Oxim überführt und dieses mit einem Dehydratisierungsreagens zur Reaktion gebracht wird.

14. Verwendung der Verbindungen nach den Ansprüchen 11 und 12 als Riechstoffe zur Herstellung von Parfüms oder Parfümbasen zur Parfümierung von Verbrauchsartikeln.

## Claims

1. 5,6,7,8-Tetrahydro-3,5,5,6,7,8,8-heptamethyl-2-naphthalenecarbonitrile, as a racemate or in the form of one of its optically active isomers.

2. Trans-5,6,7,8-tetrahydro-3,5,5,6,7,8,8-heptamethyl-2-naphthalenecarbonitrile.

3. (-)-(6S,7S)-5,6,7,8-Tetrahydro-3,5,5,6,7,8,8-heptamethyl-2-naphthalenecarbonitrile.

4. (+)-(6R,7R)-5,6,7,8-Tetrahydro-3,5,5,6,7,8,8-heptamethyl-2-naphthalenecarbonitrile.

5. Use of 5,6,7,8-tetrahydro-3,5,5,6,7,8,8-heptamethyl-2-naphthalenecarbonitrile in racemic form, or in the form of one of its optically active isomers, as a perfuming ingredient.

6. Perfuming composition or perfumed article containing as an active ingredient 5,6,7,8-tetrahydro-3,5,5,6,7,8,8-heptamethyl-2-naphthalenecarbonitrile in racemic form or in the form of one of its optically active isomers.

7. Perfumed article according to claim 6, in the form of a perfume or a cologne, a soap, a bath or shower gel, a shampoo or other hair-care product, a cosmetic preparation, an air or body deodorant, a detergent or fabric softener, or a household product.

8. Process for the preparation of 5,6,7,8-tetrahydro-3,5,5,6,7,8,8-heptamethyl-2-naphthalenecarbonitrile, characterized by reacting the oxime of 5,6,7,8-tetrahydro-3,5,5,6,7,8,8-heptamethyl-2-naphthalenecarbaldehyde with a dehydrating agent.

9. Process according to claim 8, wherein there is used as starting product the oxime of trans-5,6,7,8-tetrahydro-3,5,5,6,7,8,8-heptamethyl-2-naphthalenecarbaldehyde to obtain trans-5,6,7,8-tetrahydro-3,5,5,6,7,8,8-heptamethyl-2-naphthalenecarbonitrile.

10. Oxime of 5,6,7,8-tetrahydro-3,5,5,6,7,8,8-heptamethyl-2-naphthalenecarbaldehyde.

11. (-)-(6S,7S)-5,6,7,8-Tetrahydro-3,5,5,6,7,8,8-heptamethyl-2-naphthalenecarbaldehyde.

12. (+)-(6R,7R)-5,6,7,8-Tetrahydro-3,5,5,6,7,8,8-heptamethyl-2-naphthalenecarbaldehyde.

13. Use of the compounds according to claims 11 and 12 as starting products for the preparation of (-)-(6S,7S)-5,6,7,8-tetrahydro-3,5,5,6,7,8,8-heptamethyl-2-naphthalenecarbonitrile, respectively (+)-(6R,7R)-5,6,7,8-tetrahydro-3,5,5,6,7,8,8-heptamethyl-2-naphthalenecarbonitrile, characterized in that the starting product is converted, in a generally known manner, into the corresponding oxime and the latter oxime is subsequently reacted with a dehydrating agent.

14. Use of the compounds according to claims 11 and 12 as perfuming ingredients for the preparation of perfumes and perfuming bases and for perfuming consumer products.